# EUROPEAN PATENT APPLICATION

(11) **EP 4 545 523 A1**
(43) Date of publication of application: **30.04.2025**
(21) Application number: 23826454.3
(22) Date of filing: 20.06.2023
(51) Int. Cl.: C07D 413/12

(54) **PREPARATION PROCESS FOR JAKTINIB DIHYDROCHLORIDE MONOHYDRATE**

(30) Priority: 21.06.2022 CN 202210707468
(71) Applicant: Suzhou Zelgen Biopharmaceutical Co., Ltd., Jiangsu 215300 (CN); Shanghai Zelgen Pharma.Tech Co., Ltd., Shanghai 201203 (CN)
(72) Inventor: LV, Binhua, Shanghai 201203 (CN); LI, Chengwei, Shanghai 201203 (CN); DING, Yingcai, Shanghai 201203 (CN); PANG, Xudong, Shanghai 201203 (CN); GUO, Chao, Shanghai 201203 (CN)
(74) Representative: dompatent von Kreisler Selting Werner - Partnerschaft von Patent- und Rechtsanwälten mbB
(86) International application number: PCT/CN2023/101490
(87) International publication number: WO 2023/246807

(57) **Abstract**

The present invention relates to a preparation method for a high-purity Jaktinib dihydrochloride monohydrate compound represented by formula (A). The whole process does not comprise special reaction conditions and complex post-treatment processes, and is easily available in raw materials and reagents, high in conversion rate, environment-friendly, and very suitable for industrial production.

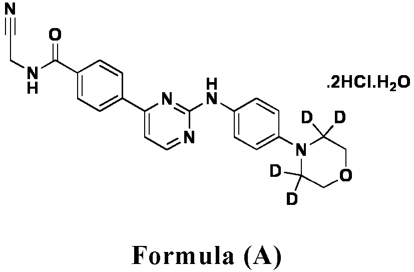

## Description

### Technology Field

This invention relates to the field of medicine. In particular, the present invention relates to a method for the preparation and purification of Jaktinib dihydrochloride monohydrate.

### Background Technology

### N-(cyanomethyl)-4-(2-((4-(morpholino-3,3,5,5-d₄)phenyl)amino)pyrimidin-4-yl)benzamide, its structure is shown in the formula (F):

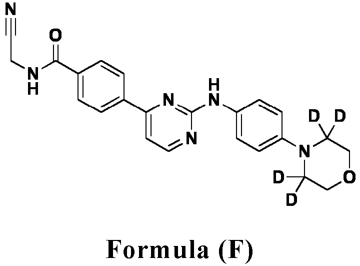

The molecular formula of the compound of the formula (F) is C₂₃H₁₈D₄N₆O₂ with a molecular weight of 418.49, which is a class of receptor tyrosine kinase inhibitors such as JAKi for the preparation of drugs for the treatment or prevention of cancer, myeloproliferative neoplasm, inflammation, immunity and other related diseases.

In patent WO2014114274A1, the synthetic route of the formula (F) from 4-(2-chloropyrimidine-4-yl)methyl benzoate and 4-(morpholine-3,3,5,5-*d*₄)aniline via S_{N}Ar substitution, hydrolysis, amide condensation and hydrochloric acid salt formation is disclosed, however, the S_{N}Ar substitution reaction was catalyzed by *p-*toluenesulfonic acid monohydrate, which brought mutagenic impurities of sulfonate After hydrolysis, the free acid particles are neutralized by acid and are too fine to be filtered; EDCI/HOBt condition is used to form many by-products during condensation, which is difficult to purify. In addition, methanol was both used in the hydrolysis step and preparing 4-(morpholinyl-3,3,5,5-*d*₄) aniline, resulting in lower yield and more by-products, not suitable for industrial production.

Therefore, the field still needs to develop a better method for the synthesis of Jaktinib hydrochloride.

### The content of the invention

The aim of this invention is to provide a synthetic method for preparing Jaktinib dihydrochloride monohydrate and its intermediates, which is low-cost, safe, environment-friendly, high yield and is more suitable for industrial production.

The first aspect of this invention provides a method for preparing the compound shown in the formula (A), wherein, the method comprises the following steps: 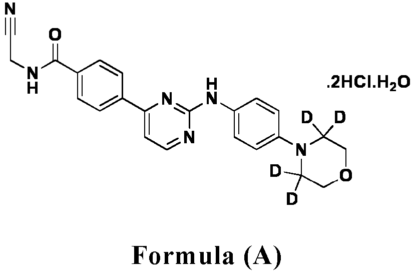
(a) In a selected solvent with basic activator and condensation reagent, the compound of the formula (E) or its hydrate reacts with acetonitrile or its salt to obtain the compound of the formula (F).
   (b) 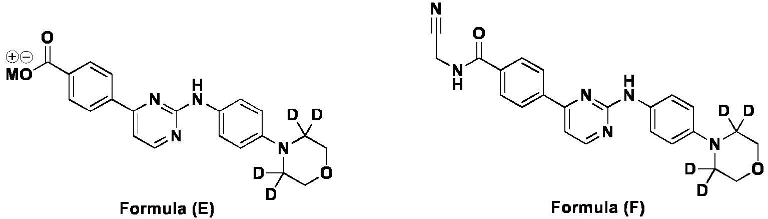 Wherein, M = alkali metal ions or alkaline metal ions;
(b) In a selected solvent, the compound of the formula (F) reacts with hydrochloric acid to obtain the hydrochloride of the formula (F);
(c) In a selected solvent, the hydrochloride polymorph of the formula (F) is transformed, and the compound of the formula (A) is obtained.

In another preferred embodiment, M = Li, Na or K_{∘}

In another preferred embodiment, the salt of acetonitrile is acetonitrile hydrochloride.

In another preferred embodiment, step (a) may also comprise the step of recrystallizing the crude product of the compound of the formula (F) in a solvent selected preferably from dimethyl sulfoxide, N,N-dimethylformamide, acetone, methanol, ethanol, ethyl acetate or a mixture thereof, preferably in a mixture of dimethyl sulfoxide and ethanol.

In another preferred embodiment, in step (a), the crude compound of the formula (F) recrystallizes in a solvent to obtain a refined product, the crude product is firstly thermally dissolved in dimethyl sulfoxide, and then dropped into the resulting hot filtrate with ethanol, which is cooled and stirred, a high purity free alkali compound of the formula (F) was obtained, with the weight ratio of dimethyl sulfoxide to the formula (E) being 1:10 to 10:1, preferably 1:4 to 4:1, and the temperature range of hot solution being 60 to 100 °C, preferably 75 to 85 °C The weight ratio of ethanol to the compound of the formula (E) is 1:10 to 10:1, preferably 1:2 to 5:1.

In another preferred embodiment, in step (a), the solvent is selected from dimethyl sulfoxide, dichloromethane, methanol, ethanol, oxolane, acetone, N-methyl pyrrolidone, N,N-dimethylacetamide, N,N-dimethylformamide, or a combination thereof.

In another preferred embodiment, in step (a), the basic activator is selected from triethylamine, pyridine, dimethylaminopyridine, potassium carbonate, pyridino-triazole, N,N-diisopropylethylamine, or a combination thereof, preferably N,N-diisopropylethylamine.

In another preferred embodiment, in step (a), the reaction temperature is -30 to 50°C, preferably -15 to 30°C, preferably -10 to 15°C.

In another preferred embodiment, in step (a), the condensation reagent is selected from CDI (*N*,*N*'-carbonyldiimidazol), DCC (*N*,*N*'-dicyclohexylcarbodiimide), HATU (2-(7-Azabenzotriazol-1-yl)-*N,N,N',N'*-tetramethyluronium hexafluorophosphate), HBTU (O-benzotriazol-1-yl-tetramethyluronium hexafluorophosphate), TBTU (O-(benzotriazol-1-yl)-*N,N,N,N*-tetramethyluronium tetrafluoroborate), Propylphosphonic anhydride solution (T3P), BOP ((Benzotriazollyloxy)tris(dimethylamino)phosphonium hexafluophosphate) and PyBOP (Benzotriazol-1-yl-oxytripyrrolidino-phosphonium hexafluorophosphate). Preferably, the condensation reagent is PyBOP.

In another preferred embodiment, in step (b), the solvent is selected from dimethyl sulfoxide, ethyl acetate, ethanol, oxolane, N-methyl pyrrolidone, N,N-dimethylformamide and acetone or their mixture. Preferably, the solvent is a mixture of dimethyl sulfoxide and acetone.

In another preferred embodiment, the weight ratio of dimethyl sulfoxide and acetone is 1:20 to 20:1, preferably 1:10 to 10:1, and preferably 1:5 to 5:1.

In another preferred embodiment, in step (b), the weight ratio of hydrochloric acid to the compound of the formula (F) is 1:10 to 10:1, preferably 1:5 to 5:1, and preferably 1:3 to 3:1.

In step (c), the time of crystal transformation is 0.5 to 36 hours, preferably 1 to 18 hours, and preferably 2 to 9 hours.

In another preferred embodiment, in step (c), the resulting compound of the formula (A) has a purity more than 99.0%, preferably more than 99.5%, and more preferably more than 99.7%.

In another preferred embodiment, in step (c), the solvent used for the polymorph transformation is selected from dioxane, oxolane, acetonitrile, acetone and water or their mixture, the preferred solvent is a mixture of acetone and water.

In another preferred embodiment, in step (c), the weight ratio of the mixed solvent is 1:50 to 50:0.1, preferably 1:25 to 25:0.2, and preferably 1:10 to 10:0.5.

In another preferred embodiment, in step (c), the resulting compound of the formula (A) has an isomer-related impurity (G) less than 0.10%, preferably, less than 0.08%, and more preferably, less than 0.05%.

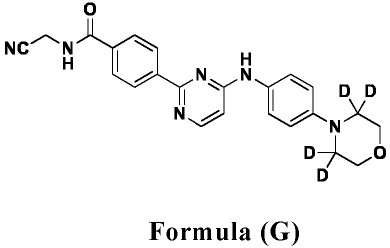

In another preferred embodiment, in step (c), the content of the relevant impurity of the formula (H) in the resulting compound of the formula (A) is less than 0.15%, preferably less than 0.07%, preferably less than 0.05%, and preferably undetected.

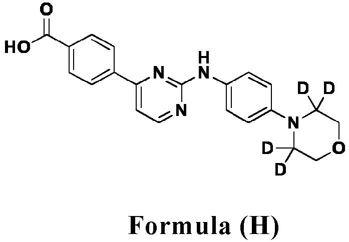

In another preferred embodiment, in step (c), the resulting compound of the formula (A) contains less than 0.10% of the relevant impurity of the formula (D), preferably, less than 0.06%, preferably, less than 0.05%, preferably undetected.

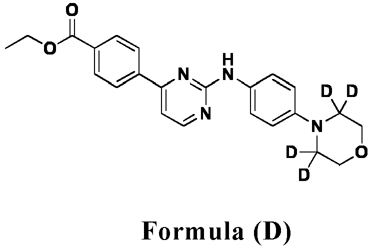

In another preferred embodiment, the compound of the formula (E) or its hydrate is prepared as follows:
a1) In the presence of alkali in selected solvent, the compound of the formula (D) undergoes hydrolysis and is filtered directly to obtain the compound of the formula (E) or its hydrate.

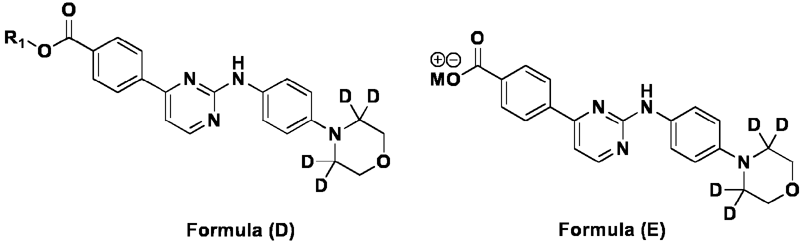

Wherein, R₁ is C1-C6 alkyl, preferably C1-C3 alkyl, more preferably methyl or ethyl. M is defined as described above.

In another preferred embodiment, in step a1), the base is selected from sodium hydroxide, potassium hydroxide and lithium hydroxide or their hydrates, preferably lithium hydroxide monohydrate.

In another preferred embodiment, in step a1), the solvent is selected from methanol, ethanol, isopropanol, dioxane, oxolane, water, or their mixture, preferably a mixture of ethanol, oxolane and water.

In another preferred embodiment, in step a1), the reaction temperature is 40 to 100°C, preferably 60 to 80°C.

In another preferred embodiment, the compound of the formula (D) is prepared as follows:
a0) In a solvent, in the presence of acid or in a pure solvent without acid, compound (B) reacts with the formula (C) to obtain the salt of compound (D) , which is then pH-adjusted to neutral or alkaline by alkali to obtain compound (D).

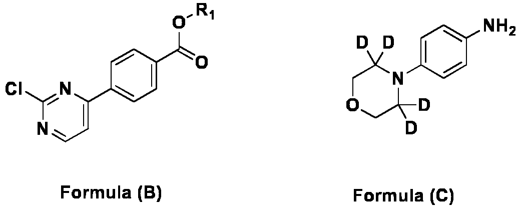

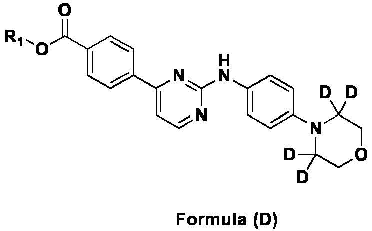

Wherein, R₁ is defined as described above.

In another preferred case, step a0) also comprises a work up step including filtration or centrifugation, rinsing with purified water, and drying.

In another preferred example, in step a0), in the presence of an acid in a solvent, the compound of the formula (B) reacts with formula (C) to obtain a salt of the compound of the formula (D), which is then adjusted by base to pH 8-10, preferably 8-9, to obtain the compound (D), preferably including a work up step.

a0-1) The purified compound (D) is obtained by centrifugation, washing and drying with purified water. Preferably, the purity of compound (D) is more than 95% and the yield is more than 70%.

In another preferred example, in step a0), the solvent is selected from dimethyl sulfoxide, *N,N-dimethylformamide,* methanol, ethanol, isopropanol, *tert*-butanol, n-pentanol, isobutanol, sec-butanol, *tert*-butanol, oxolane, acetone, acetonitrile, dioxane, or a mixture thereof, preferably from *tert*-butanol, isopropanol, dioxane, more preferably dioxane.

In another preferred embodiment, in step a0), the reaction temperature is 40 to 150°C, preferably 70 to 120°C, more preferably 85 to 105°C.

In another preferred embodiment, in step a0), the acid is selected from hydrochloric acid, phosphoric acid, formic acid, benzene sulfonic acid, citric acid, boron trifluoride ether complex, benzene sulfonic acid, and *p*-toluenesulfonic acid monohydrate, preferably *p*-toluenesulfonic acid monohydrate.

In another preferred embodiment, in step a0), in a pure solvent without acid, where the pure solvent is selected from N-amyl alcohol, iso-butanol, sec-butanol, tert-amyl alcohol, or a combination thereof, preferably, the pure solvent is selected from sec-butanol and tert-amyl alcohol, preferably tert-amyl alcohol.

In another preferred embodiment, in step a0), the base is selected from sodium hydroxide, potassium hydroxide, triethylamine, sodium bicarbonate, sodium carbonate, and potassium carbonate, preferably sodium carbonate or potassium carbonate.

In another preferred example, the compound of the formula (E) is selected from 4-(2-(4-(morpholine-3,3,5,5-*d*₄)phenyl)pyrimidine-4-yl)benzoic acid lithium monohydrate, 4-(2-((4-(morpholine-3,3,5,5-*d*₄)phenyl)pyrimidine-4-yl)benzoic acid sodium salt, 4-(2-((4-(morpholine-3,3,5,5-*d*₄)phenyl) pyrimidine-4-yl)benzoic acid potassium salt. Preferably a lithium 4-(2-(4-(morpholine-3,3,5,5-*d*₄)phenyl)amino)pyrimidine-4-yl) benzoate monohydrate.

The second aspect of the invention provides a compound or hydrate thereof as shown in the formula (E) below.

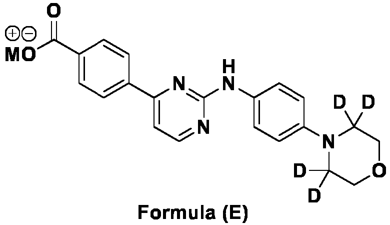

Wherein, M = alkali metal ion or alkaline earth metal ion.

In another preferred example, M = Li, Na or K_{∘}

In another preferred example, the compound is

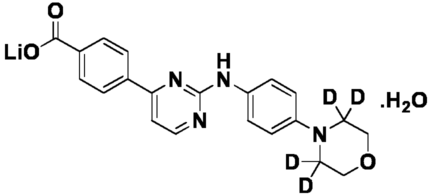

The third aspect of the invention provides a use of the compound of the formula (E) as described in the second aspect in the synthesis of the following compound of the formula (J).

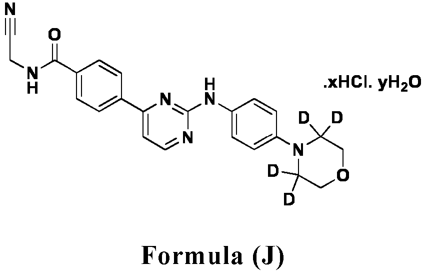

Wherein, x = 0, 1 or 2; y = 0 or 1.

It should be understood that in the scope of this invention, the above-mentioned technical features of the invention and those described in detail below, such as embodiments, may be combined with each other, thus constituting a new or preferred technical scheme. Limited to the length, here no longer repeated one by one.

### Detailed implementation methods

After a long-term and in-depth study, the invention develops a new preparation method of Jaktinib dihydrochloride monohydrate, which has the advantage of low cost, safe process, environment-friendly and high yield, more suitable for industrial production. On this basis, the invention is completed.

### Terms

### Intermediate

Intermediate refers to semi-finished product, is the production of products needed in the process of formation. Generally, an inventor can produce a product from an intermediate as a starting material. Therefore, screening suitable intermediates can optimize the process route, and then achieve the goal of increasing yield, saving time and cost.

In this invention, the intermediate means the following compound of the formula (E) or its hydrate thereof.

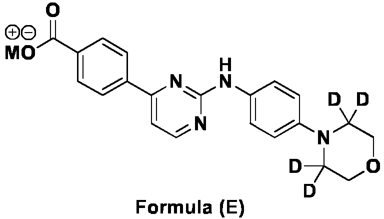

Wherein, M = alkali metal ion or alkali earth metal ion, preferably M = Li, Na or K, more preferably M = Li.

Preferably, the intermediate is

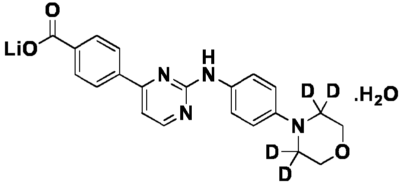

### Method for preparing the intermediate

The preparation method of the intermediate comprises the steps of:
The compound of the formula (D) is hydrolyzed at a certain temperature (such as 40 to 100°C, the optimum temperature is 60 to 80°C) in a solvent (For example, methanol, ethanol, isopropyl alcohol, dioxane, oxolane, water or their mixed solvents, preferably ethanol, oxolane and water) under the action of alkali (such as sodium hydroxide, potassium hydroxide and lithium hydroxide or their hydrates, preferred is lithium hydroxide hydrate) to obtain the carboxylate or hydrate of the formula (E). 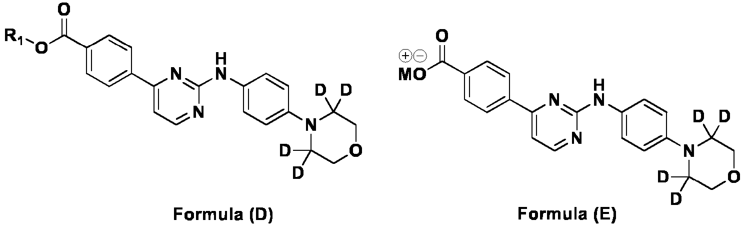
Wherein, R₁ is C1-C6 alkyl, preferably C1-C3 alkyl, preferably methyl and ethyl;
M is defined as above.

Preferably, the method also includes steps

Compound (B) and compound (C) are heated (such as 40 to 150°C, preferably 70 to 120°C, more preferably 85 to 105°C) in a selected solvent (Dimethyl sulfoxide, N,N-dimethylformamide, methanol, ethanol, isopropanol, tert-butanol, n-pentanol, iso-butanol, sec-butanol, tert-pentanol, oxolane, acetone, acetonitrile, dioxane, preferably tert-butanol, dioxane, more preferably dioxane) by acid catalysis (hydrochloric acid, phosphoric acid, formic acid, benzene sulfonic acid, citric acid, boron trifluoride ether complex, benzene sulfonic acid, *p*-toluenesulfonic acid monohydrate, preferred is *p*-toluenesulfonic acid monohydrate) or in a pure solvent (monohydrate, n-butanol, sec-butanol, and tert-pentanol, preferably tert-butanol) to obtain salts of compound (D), which is neutralized by alkali (such as sodium hydroxide, potassium hydroxide, triethylamine, Sodium bicarbonate, sodium carbonate, and potassium carbonate, preferably sodium carbonate or potassium carbonate) and precipitated in the solvent, the resulting compound (D).

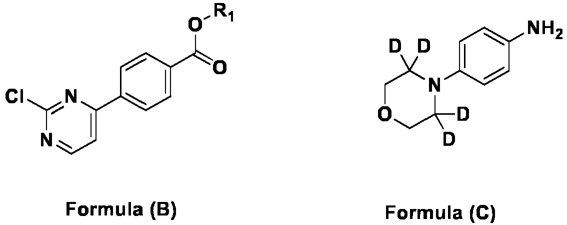

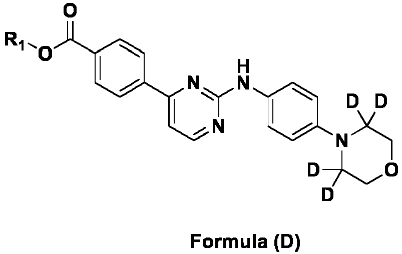

Wherein, R₁ is defined as described above.

### Method for preparing Jaktinib dihydrochloride monohydrate (compound A)

Preferably, the method includes following steps
(a)The compound of the formula (E) or its hydrate thereof, optionally obtained by condensation of a condensation reagent (such as PyBOP) with acetonitrile or its hydrochloride in a solvent (such as dimethyl sulfoxide, dichloromethane, methanol, ethanol, oxolane, acetone, N-methylpyrrolidone, N,N-dimethylacetamide and N,N-dimethylformamide, preferably N-methyl pyrrolidone and N,N-dimethylformamide, more preferably N,N-dimethylformamide), an alkaline activator (such as triethylamine, pyridine, dimethylaminopyridine, potassium carbonate, pyridino-triazole, N,N-diisopropylethylamine, preferably N,N-diisopropylethylamine) and at a suitable temperature (such as -30 to 50°C, preferably -15 to 30°C, preferably -10 to 15°C). Formula (F) crude compound recrystallizes in solvent (such as dimethyl sulfoxide, N,N-dimethylformamide, acetone, methanol, ethanol, ethyl acetate or their mixture, preferably dimethyl sulfoxide and ethanol) to obtain the refined product. 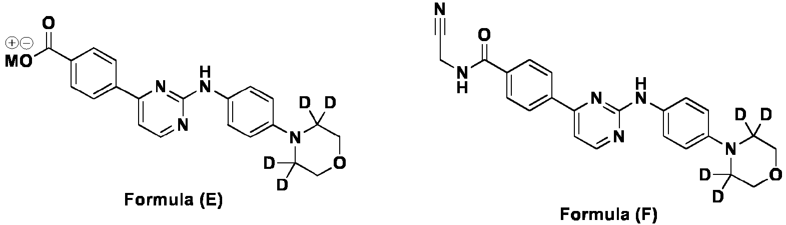 Wherein, M = alkali metal ion or alkali earth metal ion, preferably M = Li, Na or K, more preferably M = Li.
(b) A hydrochloride salt of the formula (F) which is formed by a compound in a selected solvent (dimethyl sulfoxide, ethyl acetate, ethanol, oxolane, N-methyl pyrrolidone, N,N-dimethylformamide, acetone or their mixture, preferably a mixture of dimethyl sulfoxide and acetone, the ratio of the mixed solvent is 1:20 to 20:1 (m/m, relative to the formula (F)), preferably 1:10 to 10:1, especially preferably 1:5 to 5:1) in a suitable proportion and a hydrochloric acid (such as 1:10 to 10:1, m/m, relative to the formula (F), preferably 1:5 to 5:1, especially preferably 1:3 to 3:1) in a suitable proportion;
(c) The hydrochloride salt is then subjected to a suitable solvent ratio (such as dioxane, tetrahydrofuran, acetonitrile, acetone, water or a mixed system thereof, preferably a mixture of acetone and water; the mixed solvent ratio is 1:50 to 50:0.1 (m/m, relative to the formula (F)), preferably 1:25 to 25:0.2, especially preferably 1:10 to 10:0.5) for a certain period of time (such as 0.5 to 36 hours, preferably 1 to 18 hours, especially preferably 2 to 9 hours) Stable acquisition of the compound of the formula (A) through polymorph transformation;

For purification, the crude product is firstly dissolved in a certain proportion of dimethyl sulfoxide, and then a certain proportion of ethanol is dropped into the resulting hot filtrate, which is cooled and stirred to obtain a high purity of free alkali compound of the formula (F).

Preferably, for refining, the selected dimethyl sulfoxide ratio is 1:10 to 10:1 (m m, relative to the formula (E)), preferably 1:4 to 4:1. The selected temperature range is 60 to 100°C, preferably 75 to 85 °C. The ratio of ethanol is 1:10 to 10:1 (m/m, relative to the formula (E)), 1:2 to 5:1 is preferred.

In detail, this process comprises the following steps: (1) Using compound 4-(2-chloropyrimidine-4-yl)ethyl benzoate of the formula (B) and compound 4-(morpholine-3,3,5,5-*d*₄)aniline of the formula (C) as major starting materials, the intermediate of the formula (D) was obtained by S_{N}Ar substitution reaction. (2) The compound of the formula (D) was hydrolyzed by reflux under alkaline condition and then filtered directly to obtain its carboxylate of the formula (E). (3) The active intermediate of the formula (F) is obtained by the condensation of compound of the formula (E) and amino acetonitrile hydrochloride via amide condensation. (4) The compound of the formula (F) and hydrochloric acid were salted to obtain Jaktinib dihydrochloride monohydrate, which was stably obtained by crystallization in proper solvent.

Preferably, the process also includes the preparation of the intermediate of the formula (E).

Compared with the current art, this preparation method of the invention has a series of advantages. The major advantage includes:
(1) All intermediates obtained in each step of the invention are in solid form and are easy to purify, separate and store.
(2) Compared with the current art, this process of the invention contains no solvent extraction, and is purified by centrifugal filtration, crystallization, etc.
(3) Compared with the current art, the invention obtains the corresponding carboxylate intermediate (especially the lithium salt intermediate) with higher purity, avoids charging strong acid, and the obtained solid is easy to filter, economic and environmental-friendly.
(4) Compared with the current art, the PyBOP condensation reagent is adopted, leading to faster reaction, obviously reduced by-product, simpler work up, higher yield, and with easier recrystallization for purification.
(5) Compared with the current art, the synthetic route of the present invention has a higher total yield of 64% with better atom economy.
(6) Compared with the current art, the obtained Jaktinib dihydrochloride monohydrate of the present invention has higher purity and less or undetected content of specific related substances.

The following will further elaborate on the present invention in conjunction with specific embodiments. Which should be understood is that these embodiments are only used to illustrate the present invention and not to limit the scope of the present invention. The experimental methods in the following embodiments that do not specify specific conditions are usually based on conventional conditions or conditions recommended by the manufacturer. Unless otherwise specified, percentages and portions are calculated by weight.

### Example 1 Preparation of N-(cyanomethyl)-4-(2-((4-(morpholine-3,3,5,5-d4)phenyl)amino)pyrimidin-4-yl) benzamide dihydrochloride monohydrate (Compound 6, compound of formula (A))

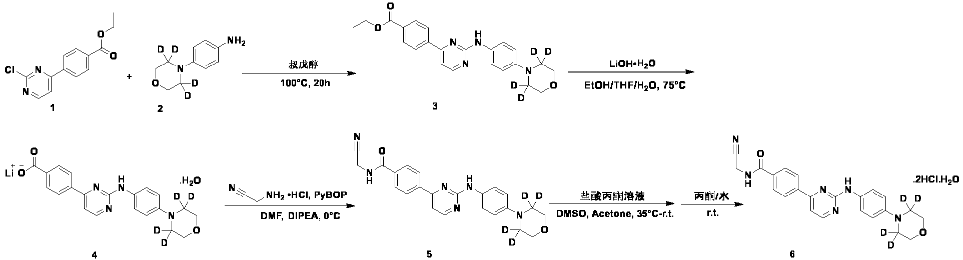

### 1. Preparation of 4-(2-((4-(morpholino-3,3,5,5-d4)phenyl)amino)pyrimidin-4-yl)ethyl benzoate (Compound 3)

*Tert*-butanol (523.90kg), 4-(2-chloropyrimidin-4-yl)ethyl benzoate (52.85kg) and 4-(morpholino-3,3,5,5-d4)aniline (35.00kg) was subsequently added to a 3000L reactor at room temperature under nitrogen protection. The mixture was held stirring until the reaction pass, cool down to room temperature and keep stirring for another 3 hours, filtration and the resulting cake was washed with tert-butanol. The wet cake was suspended in purified water (840.55kg), then 25% potassium carbonate solution was dropped for dissociation. Keep stirring for another 2 hours, filtration and the cake was rinsed twice with purified water. 67.88kg of the title compound was prepared by vacuum drying below 70 degree with a purity of 99.2% and a yield of 87%.

¹H NMR (400MHz, DMSO-*d*₆) δ: 9.54 (s, 1H), 8.55 (d, J=4.0Hz, 1H), 8.29 (d, J=8.0Hz, 2H), 8.11 (d, J=8.0Hz, 2H), 7.66-7.68 (dd, J=8.0Hz, 2H), 7.40 (d, J=8.0Hz, 1H), 6.93-6.95 (dd, J=8.0Hz, 2H), 4.33-4.39 (q, J=8.0, 16.0Hz, 2H), 3.74 (s, 4H), 1.36 (t, J=8.0Hz, 3H).

¹³C NMR (400MHz, DMSO-*d*₆) δ: 14.63, 48.54-49.23, 61.44, 66.52, 108.17, 116.02, 120.86, 127.58, 130.05, 132.03, 133.21, 141.58, 146.76, 159.83, 160.85, 162.79, 165.79.

LC-MS: 409.1 (M+H)⁺ _{∘}

### 2. Preparation of 4-(2- ((4-(morpholino-3,3,5,5-d4)phenyl) amino)pyrimidin-4-yl)benzoate lithium salt monohydrate (Compound 4)

Alcohol (159.85kg), lithium hydroxide monohydrate, compound 3 (68.50kg), water (139.30kg) and tetrahydrofuran (61.50kg) was subsequently added to a 500L reactor at room temperature under nitrogen protection. The mixture was warmed to 75 degree and hold stirring for 16 hours, cool down to room temperature and keep stirring for another 1 hour before filtration. 62.30kg of the title compound was prepared by vacuum drying below 60 degree with a purity of 99.9% and 93% yield.

¹H NMR (400MHz, DMSO-*d*₆) δ: 9.52 (s, 1H), 8.54 (d, J=4.0Hz, 1H), 8.26 (d, J=8.0Hz, 2H), 8.10 (d, J=8.0Hz, 2H), 7.67 (d, J=8.0Hz, 2H), 7.38 (d, J=4.0Hz, 1H), 6.93 (d, J=8.0Hz, 2H), 3.73 (s, 4H).

¹³C NMR (400MHz, DMSO-*d*₆) δ: 48.93, 66.52, 108.16, 116.04, 120.85, 127.46, 130.23, 133.15, 133.24, 141.22, 146.75, 159.77, 160.87, 162.98, 167.40.

LC-MS: 381.1 (M+H)⁺ _{∘}

Element analysis: C=61.98%, N=13.80%. Calculated based on the presence of one molecule of crystalline water, the theoretical value of C and N in compound 4 is 62.37% and 13.86%, respectively.

### 3. Preparation of N-(cyanomethyl)-4-(2-((4-(morphinyl-3,3,5,5-d4)phenyl)amino)pyrimidin-4-yl) benzamide (Compound 5)

DMF (528.20kg), compound 4 (61.88kg), benzotriazol-1-yl-oxytripyrrolidinophosphonium hexafluorophosphate (PyBOP) and amino acetonitrile hydrochloride and N,N-diisopropylethylamine was subsequently added to a 1000L reactor under nitrogen protection. The mixture was held stirring within 10 to 0°C. After the reaction pass, the reaction solution was added dropwise to a 5000L reactor with pre-added water (2000.00kg), keep stirring for another 3 hours at room temperature, filtration and the resulting cake was rinsed with water (600.45kg). The wet cake was dried over below 65°C, and the resulting crude product was added to a 1000L reactor, followed with DMSO (120.00kg), keep stirring at 80°C until completely dissolving, then ethanol (250kg) was dropped. The mixture was stirred for another 2 hours around 80°C before cooling to room temperature, filtration and the cake was washed with ethanol. 57.48kg of title compound was prepared by vacuum drying below 60 degree with a purity of 99.6% and 90% yield.

¹H NMR (400MHz, DMSO-*d*₆) δ: 9.51 (s, 1H), 9.36 (t, J=4.0Hz, 1H), 8.54 (d, J=8.0Hz, 1H), 8.28 (dd, J=8.0Hz, 2H), 8.04 (dd, J=8.0Hz, 2H), 7.68 (dd, J=8.0Hz, 2H), 7.40 (d, J=8.0Hz, 1H), 6.93 (dd, J=8.0Hz, 2H), 4.38 (d, J=8.0Hz, 2H), 3.73 (s, 4H).

¹³C NMR (400MHz, DMSO-*d*₆) δ: 28.27, 48.88, 66.51, 108.09, 116.00, 118.09, 120.87, 127.43, 128.40, 133.23, 135.01, 140.45, 146.74, 159.74, 160.84, 162.91, 166.63.

LC-MS: 419.1 (M+H)⁺_{∘}

### 4. Preparation of N-(cyanomethyl)-4-(2-((4-(morphinyl-3,3,5,5-d4)phenyl)amino)pyrimidin-4-yl)benzamide dihydrochloride monohydrate (Compound 6)

DMSO (286.65kg) and compound **5** (56.10kg) was subsequently added to a 1000L reactor under nitrogen protection. The mixture was held stirring until dissolved, filtration and rinsed with DMSO (24.50kg). The combined filtrate was add pre-prepared hydrochloride acetone solution (30.00kg HCl added with 527.0kg acetone within -5 to 5°C). The mixture was stirred for 2 hours before filtration, and the resulting cake was washed with acetone. Adding the wet cake to another reactor, followed with acetone and water. The salt was held slurring until the desired polymorph formed completely, filtration and rinsing with acetone. 61.00kg of title compound was prepared by vacuum drying around 55 degree with KF of 3.8%, a purity of 99.9%, impurity G of 0.05%, impurity H of 0.02%, impurity D not detected and 90% yield.

¹H NMR (400MHz, DMSO-*d*₆) δ: 10.19 (s, 1H), 9.64 (t, J=4.0Hz, 1H), 8.64 (d, J=8.0Hz, 1H), 8.28 (d, J=8.0Hz, 2H), 8.10 (d, J=8.0Hz, 2H), 7.98 (d, J=8.0Hz, 2H), 7.87 (d, J=8.0Hz, 2H), 7.57 (d, J=4.0Hz, 1H), 6.70 (brs, 4H), 4.35 (d, J=8.0Hz, 2H), 4.12 (s, 4H).

¹³C NMR (400MHz, DMSO-*d*₆) δ: 28.26, 153.95, 63.83, 109.57, 1118.08, 120.09, 122.29, 127.65, 128.55, 135.33, 136.36, 139.83, 141.34, 159.09, 159.72, 163.72, 166.49.

LC-MS: 419.1 (M+H)⁺_{∘}

### Example 2 Preparation of N-(cyanomethyl)-4-(2-((4-(Morphinyl-3,3,5,5-d4)phenyl)amino)pyrimidin-4-yl)benzamide dihydrochloride monohydrate (Compound 6, compound of formula (A))

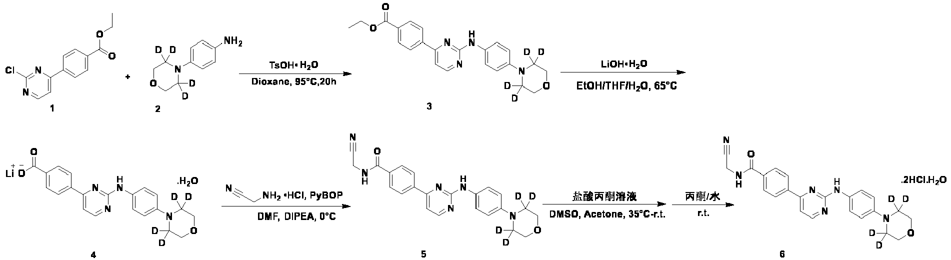

### 1. Preparation of 4-(2-((4-(morpholino-3,3,5,5-d4)phenyl)amino) pyrimidin-4-yl) ethyl benzoate (Compound 3)

1,4-dioxane (20.60 kg) was added to a 50L jacket reactor, start stirring and sequentially adding 4- (2-chloropyrimidin-4-yl) ethyl benzoate (1513 g, compound 1), 4-(morpholin-3-3,5,5-d₄₋)aniline (1000 g, compound **2),** and *p*-toluenesulfonic acid monohydrate (938 g). Heat to control the internal temperature to about 95°C and hold stirring for 20 hours. After the IPC pass, cool down to room temperature, centrifugation and the filter cake was rinsed with 1,4-dioxane. After sufficient dryness, the resulting solid was added to another reactor with remaining purified water (20.00 kg), stirring and adding potassium carbonate aqueous solution to adjust the pH to 8-9, centrifuge, rinsing with purified water (12.00 kg), and drying to obtain 3.40 kg of wet product which is directly used for the next reaction without further purification. The molar yield of the reaction is calculated to be 74%.

### 2. Preparation of 4-(2-((4-(morpholino-3,3,5,5-d4)phenyl)amino) pyrimidin-4-yl) benzoate lithium salt monohydrate (Compound 4)

Add compound 3 (1.66 kg), anhydrous ethanol (3.86 kg), and tetrahydrofuran (1.47 kg) to a 20L reactor in sequence, start stirring, then lithium hydroxide monohydrate (427 g) and purified water (3.3 kg) were added. The mixture was stirred around 65°C for 16 hours. After the IPC pass, cool down to room temperature, filtration, and the filter cake was washed with anhydrous ethanol. The title compound was obtained by vacuum drying, with a purity of 99.6% and a yield of 90%.

### 3. Preparation of N - (Cyanomethyl) -4- (2- ((4- (Morphinyl-3,3,5,5-d4) phenyl) amino) pyrimidin-4-yl) benzamide (Compound 5)

DMF (13.84 kg) was charged to a 20L reactor, start stirring add compound **4** (1.47 kg) was added at around 0°C, then hexafluorophosphate benzotriazol-1-yl-oxytripyrrolyl phosphate (PyBOP), aminoacetonitrile hydrochloride and N,N-diisopropylethylamine (2.00 kg) was added slowly. The mixture was stirred keeping the internal temperature around 0°C until the IPC pass, filtration to remove insoluble solid, then the filtrate was slowly added to purified water (50.00 kg), start stirring before filtration, and the resulting cake was rinsed with purified water firstly, then ethanol (7.90 kg) was charged for further wash. Hold vacuum dry at 60°C for 18 hours to obtain the crude product. DMSO (2.85 kg) was charged to the crude solid, keep stirring at 80°C until completely dissolving, add ethanol (5.94 kg) was added by dropwise, stirring for another 2 hours before cooling to room temperature. Filtration and rinsing with ethanol. 1.32 kg of the title compound was obtained by vacuum drying at 60°C with a purity of 99% and a yield of 87%.

### 4. Preparation of N-(Cyanomethyl)-4-(2-((4-(morphinyl-3,3,5,5-d4)phenyl)amino)pyrimidin-4-yl)benzamide dihydrochloride monohydrate (compound 6)

DMSO (28.65 kg) and compound 5 (5.61 kg) was charged to a 100L glass reactor under nitrogen protection, start stirring until totally dissolving, the mixture was transferred to a clean reactor through pressure filtration, rinsing with DMSO (2.45 kg). The combined filtrate was added pre-prepared hydrochloride acetone solution (3.00kg HCl added with 52.70kg acetone within -5 to 5°C). The mixture was stirred for 2 hours before filtration, and the resulting cake was washed with acetone. Adding the wet cake to another reactor, followed with acetone and water. The salt was held slurring until the desired polymorph formed completely, filtration and rinsing with acetone. 5.95kg of the title compound was prepared by vacuum drying around 55 degree with KF of 3.7%, a purity of 99.2%, impurity G of 0.08%, impurity H of 0.09%, impurity D of 0.07% and 88% yield.

### Example 3 Preparation of N-(cyanomethyl)-4-(2-((4-(morphinyl-3,3,5,5-d4)phenyl)amino)pyrimidin-4-yl)benzamide dihydrochloride monohydrate (Compound 6, compound of formula (A))

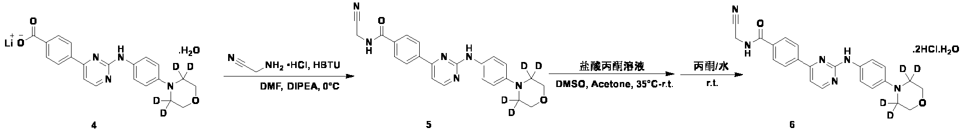

### 1. Preparation of N-(cyanomethyl)-4-(2-((4-(morphinyl-3,3,5,5-d4)phenyl)amino)pyrimidin-4-yl) benzamide (Compound 5)

DMF (13.84 kg) was charged to a 20L reactor, start stirring and adding compound **4** (1.47 kg) around 0°C, then O-benzotriazole-tetramethylurea hexafluorophosphate (HBTU), aminoacetonitrile hydrochloride and N,N-diisopropylethylamine (2.00 kg) were charged slowly. Held stirring with the internal temperature controlled around 0°C until the IPC pass, filtration to remove the insoluble solid, followed adding the filtrate to purified water (50.00 kg) slowly, start stirring and filtration, the resulting cake was washed with purified water firstly and ethanol (7.90 kg) in sequence. 1.37kg of the title compound was prepared by vacuum drying around 60 degree with a purity of 96.8% and a yield of 90%.

### 2. Preparation of N-(cyanomethyl)-4-(2-((4-(morphinyl-3,3,5,5-d4)phenyl)amino)pyrimidin-4-yl)benzamide dihydrochloride monohydrate (Compound 6)

DMSO (5.73 kg) and compound **5** (1.12 kg) were charged to a 20L glass reactor under nitrogen protection, start stirring until totally dissolving, the mixture was transferred to a clean reactor through pressure filtration, rinsing with DMSO (0.49 kg). The combined filtrate was add pre-prepared hydrochloride acetone solution (0.60kg HCl added with 10.54kg acetone within -5 to 5°C). The mixture was stirred for 2 hours before filtration, and the resulting cake was washed with acetone. Adding the wet cake to a 100L acid resistant reactor, followed with acetone and water. The salt was held slurring until the desired polymorph formed completely, filtration and rinsing with acetone. 1.16kg of the title compound was prepared by vacuum drying around 55 degree with KF of 3.9%, a purity of 98.4% , impurity G of 0.09%, impurity H of 0.45%, impurity D of 0.09% and 85% yield.

### Comparative example 1 Preparation of 4-(2-((4-(morpholino-3,3,5,5-d4)phenyl)amino)pyrimidin-4-yl)benzoate methyl ester (Compound 3-1)

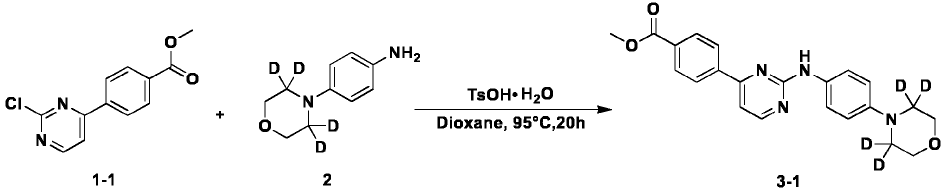

To a 5L jacketed reaction flask was added with 1,4-dioxane (2L), start stirring and sequentially adding 4-(2-chloropyrimidin-4-yl)methyl benzoate (52 g, compound 1-1), 4-(morpholine-3,3,5,5-d4)aniline (42 g, compound 2) and p-toluenesulfonic acid monohydrate (43.9 g). Heat to control the internal temperature to about 95°C and hold stirring for 20 hours. Concentration under vacuum after the IPC pass, then ethyl acetate (500mL) and 5% sodium bicarbonate solution (500mL) were charged to precipitate the solid, filtration and the obtained cake was suspended in methanol (500mL) for 5 minutes. After filtration, washing with methanol, 40g of the title compound was prepared by vacuum dry with a purity of 92% and a yield of 48%.

### Comparative example 2 Preparation of N-(cyanomethyl)-4-(2-(4- (morphinyl-3,3,5,5-d4)phenyl)amino)pyrimidin-4-yl) benzamide (Compound 5)

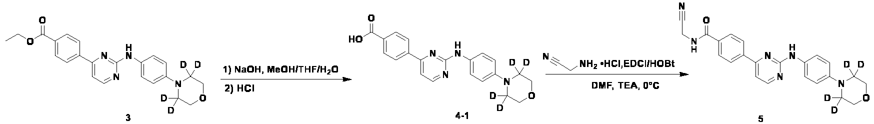

### 1. Preparation of 4-(2-((4-(morpholino-3,3,5,5-d4)phenyl)amino)pyrimidin-4-yl)benzoic acid (compound 4-1)

Compound 3 (40 g), anhydrous methanol (900 mL) and tetrahydrofuran (300 mL) were charged to a 20L reactor in sequence, start stirring, then sodium hydroxide (4.3 g) and purified water (300 mL) were added. The mixture was stirred at 65°C for 2 hours. After the IPC pass, cool down to room temperature and concentrate to remove the organic solvent. The slurry was adjusted to pH=3 with 10% dilute hydrochloric acid, filtration under high vacuum (completed in about 12 hours) and the resulting cake was washed with pure water. 32.4 kg of the title compound was obtained by vacuum dried with a purity of 99.0% and a yield of 87%.

### 2. Preparation of N-(cyanomethyl)-4-(2-((4-(morphinyl-3,3,5,5-d₄)phenyl)amino)pyrimidin-4-yl)benzamide (Compound 5)

DMF (200 mL) was charged to a 1L reaction flask, start stirring, then compound 4-1 (17 g), 1-(3-dimethylaminopropyl)-3-ethylcarbodiimide hydrochloride (10.3 g), 1-hydroxybenzotriazole (72.4 g) and triethylamine (27.1 g) were added in sequence. Keep stirring and aminoacetonitrile hydrochloride (12.4 g) was added at last. The mixture was held stirring at room temperature for 20 hours. Purified water (200 mL) and saturated sodium bicarbonate solution (200 mL) were added to the reaction solution, yellow solid precipitated, held stirring for another 30 minutes, followed with filtration and washing with pure water. 16.6 g of the title compound was obtained after drying with a purity of 94.5% and a yield of 89%.

All references mentioned in the present invention are cited as references in this application, just as each reference is cited separately. In addition, it should be understood that after reading the above lecture content of the present invention, those skilled in the art can make various modifications or modifications to the present invention, and these equivalent forms also fall within the scope of the claims attached to the present application.

## Claims

1. A method for preparing the compound shown in the formula (A), which comprises the following steps: 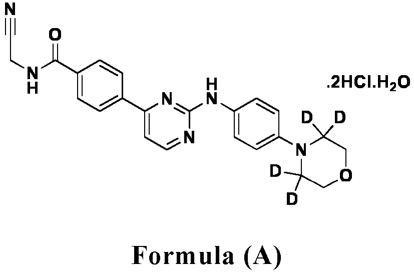
(a) In solvent, the compound of the formula (E) or its hydrate reacts with acetonitrile or its salt to obtain the compound of the formula (F) in the presence of basic activator and condensation reagent. 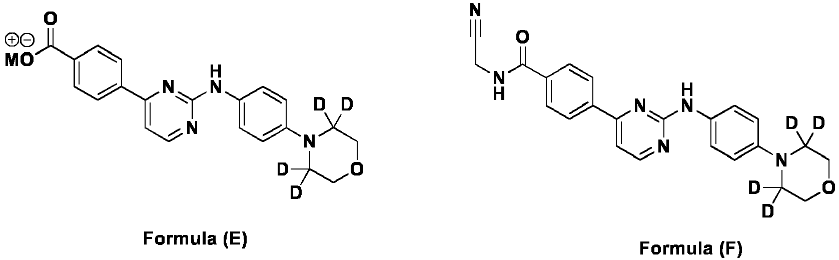 Wherein, M = alkali metal ions or alkaline metal ions.
(b) **In** solvent, the compound of the formula (F) reacts with hydrochloric acid to obtain the compound of hydrochloride of the formula (F).
(c) **In** solvent, the hydrochloride polymorph of the formula (F) is transformed into the compound of the formula (A).

2. The process of claim 1, wherein in step (a), the condensation reagent is selected from CDI (N,N'-carbonyldiimidazole), DCC (N,N'-dicyclohexylcarbodiimide), HATU (2-(7-azabenzotriazol-1-yl)-N,N,N',N'-tetramethyluronium hexafluorophosphate), HBTU (O-benzotriazol-1-yl-tetramethyluronium hexafluorophosphate), TBTU (O-(benzotriazol-1-yl)-N,N,N',N'-tetramethyluronium tetrafluoroborate), Propylphosphonic anhydride solution (T3P), BOP ((Benzotriazollyloxy)tris(dimethylamino)phosphonium hexafluophosphate) and PyBOP (Benzotriazol-1-yl-oxytripyrrolidino-phosphonium hexafluorophosphate). The preferred condensation reagent is PyBOP.

3. The process of claim 1 or claim 2, wherein in step (B), the solvent is selected from dimethyl sulfoxide, ethyl acetate, ethanol, oxolane, N-methyl pyrrolidone, N,N-dimethylformamide and acetone or their mixture. The preferred solvent is a mixture of dimethyl sulfoxide and acetone.

4. The process of any one of claims 1 to 3, wherein in step (c), the resulting compound of the formula (A) has an isomer-related impurity (G) less than 0.10%, preferably, less than 0.08%, more preferably, less than 0.05%.

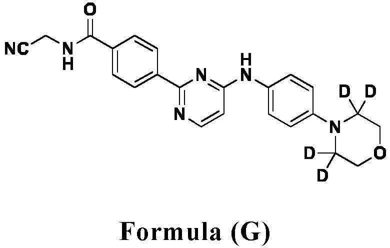

5. The method of any of claims 1 to 4, wherein in step (c), the resulting compound of the formula (A) contains less than 0.15% of the relevant impurity the formula (H), preferably, less than 0.07%, more preferably, less than 0.05%, more preferably undetected.

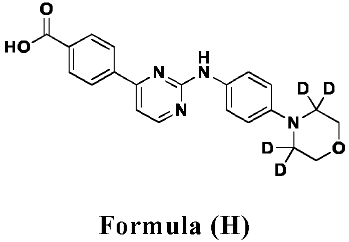

6. The method of any of claims 1 to 5, wherein in step (c), the resulting compound of the formula (A) contains less than 0.10% of the relevant impurity the formula (D), preferably less than 0.06%, more preferably less than 0.05%, more preferably undetected.

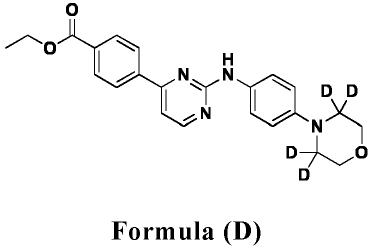

7. The method of claim 1, wherein the method for preparing the compound of the formula (E) or its hydrate is as follows:
a1) In the presence of alkali in selected solvent, the compound of the formula (D) undergoes hydrolysis and is filtered directly to obtain the compound of the formula (E) or its hydrate. 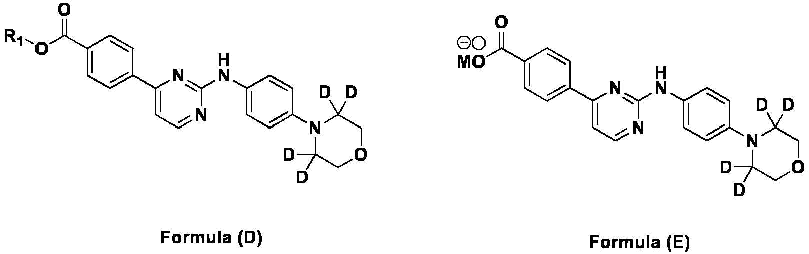 Wherein, R1 is the C1-C6 alkyl, preferably the C1-C3 alkyl, and more preferably methyl or ethyl group; M is defined as described in claim 1.

8. The method of claim 7, wherein the method for preparing the compound of the formula (D) is as follows:
a0) In a selected solvent, in the presence of an acid or in a pure solvent without any acids, the compound of the formula (B) reacts with the formula (C) to obtain the salt of the compound of the formula (D), followed by adjusting pH to neutral or alkaline by adding alkali to obtain compound (D). 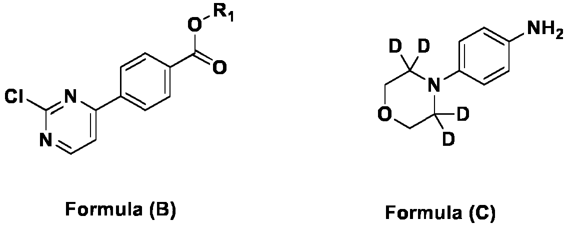 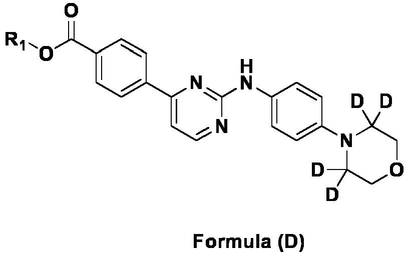 Wherein, R₁ is defined as described in claim 7.

9. The method of claim 1, wherein in step (a), the reaction temperature is -30 to 50°C, preferably -15 to 30°C, and more preferably -10 to 15°C.

10. The method of claim 1, wherein in step (a), it can also include the step of recrystallizing the crude compound of formula (F) in a solvent to obtain a purified product. Preferably, the solvent used for recrystallization is selected from dimethyl sulfoxide, N,N-dimethylformamide, acetone, methanol, ethanol, ethyl acetate or a mixture thereof. More preferably, the solvent used for recrystallization is a mixture of dimethyl sulfoxide and ethanol.

11. The method of claim 1, wherein in step (a), the step of recrystallizing the crude compound of formula (F) in a solvent to obtain the purified product is to dissolve the crude product in dimethyl sulfoxide at first, then ethanol is dropped into the obtained hot filtrate, followed with cooling and stirring to obtain a high-purity free alkali compound of formula (F). Preferably, the weight ratio of dimethyl sulfoxide to the compound of formula (E) is 1: 10 to 10:1, preferably 1:4 to 4: 1. The temperature range for hot melt is 60 to 100°C, preferably 75 to 85°C. The weight ratio of the selected ethanol to the compound of formula (E) is 1: 10 to 10:1, preferably 1:2 to 5:1.

12. The method of claim 3, wherein the weight ratio of dimethyl sulfoxide to acetone is 1:20 to 20:1, preferably 1:10 to 10:1, more preferably 1:5 to 5:1.

13. The method of claim 1, wherein in step (c), The solvent used for crystal transformation is selected from dioxane, tetrahydrofuran, acetonitrile, acetone and water or a mixture thereof, and the preferred solvent used is a mixture of acetone and water.

14. A compound or hydrate thereof as shown in the formula (E). 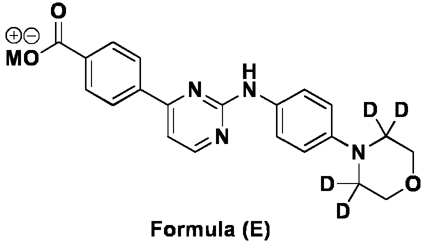 Wherein, M = alkali metal ions or alkaline earth metal ions.

15. A use of the compound of the formula E described in claim 9 in the synthesis of the following compound of the formula (J). 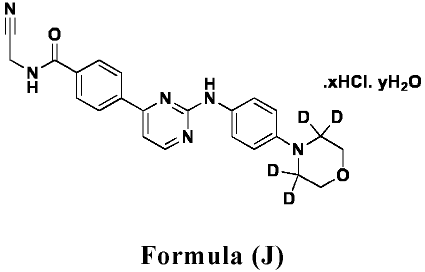 Wherein, X = 0, 1 or 2; y = 0 or 1.
